# EUROPEAN PATENT APPLICATION

(11) **EP 0 766 965 A1**
(43) Date of publication of application: **09.04.1997**
(21) Application number: 96904318.1
(22) Date of filing: 04.03.1996
(51) Int. Cl.: A61K 35/24, A61K 35/28, A61K 38/02

(54) **IMMUNOSUPPRESSIVE AGENTS**

(30) Priority: 03.03.1995 JP 68864/95
(71) Applicant: Hamajima, Fusanori, Nerima-ku, Tokyo 179 (JP); Yamakami, Kazuo, Wako-shi, Saitama 351-01 (JP)
(72) Inventor: Hamajima, Fusanori, Nerima-ku, Tokyo 179 (JP); Yamakami, Kazuo, Wako-shi, Saitama 351-01 (JP)
(74) Representative: Woods, Geoffrey Corlett
(86) International application number: JP9600513
(87) International publication number: WO9627381

(57) **Abstract**

The invention relates to an immunosuppressive agent comprising an immunosuppression-inducing protein as an active ingredient obtained from peritoneal exudates and/or spleens of mammals given cysteine protease; an immunosuppressive agent comprising as an active ingredient an immunosuppression-inducing protein against antigen, which is obtained from spleens of mammals given said antigen and cysteine protease; and an immunosuppressive agent comprising as an active ingredient an immunosuppression-inducing protein obtained by culturing macrophages contained in peritoneal exudates of mammals, in a medium containing cysteine protease.

## Description

### TECHNICAL FIELD

The present invention relates to an immunosuppressive agent used for prevention of the onset of autoimmune disease, prevention of the organ graft rejection, etc.

### BACKGROUND ART

At present, azathioprine, cyclosporin A, FK-506, 15-deoxysperguarine etc., are known as immunosuppressive agents for use in medical purposes, and they all have strong immunosuppressive effects.

However, these conventional immunosuppressive agents should be administered successively for a long time for immunosuppression because they do not induce immune tolerance. As a result, there occur unfavorable side effects such as renal ureter disturbance, blood vessel inflammation, hepatic disturbance, pancreas secretory disturbance, digestive tract disturbance, etc.

### DISCLOSURE OF INVENTION

Hence, it is the object of the present invention is to provide an immunosuppressive agent which can prevent the onset of autoimmune disease, prevention of the organ graft rejection etc. without administering it for a long time by inhibiting antibody production against antigen and transplanted organs and inducing immune tolerance against antigen and transplanted organs.

As a result of their extensive research, the present inventors found that proteins produced by macrophages or spleen of mammals upon administration of cysteine protease inhibit cellular and humoral immunity and induce immune tolerance.

That is, the present invention is an immunosuppressive agent comprising as an active ingredient immunosuppression-inducing proteins obtained from peritoneal exudates and/or spleens of mammals given cysteine protease.

Further, the present invention is an immunosuppressive agent comprising as an active ingredient immunosuppression-inducing proteins against an antigen, which is obtained from spleens of mammals given cysteine protease and said antigen.

Further, the present invention is an immunosuppressive agent comprising as an active ingredient immunosuppression-inducing proteins obtained by culturing macrophages contained in peritoneal exudates of mammals, in a medium containing cysteine protease.

Further, the present invention is cellular- and humoral-immunosuppression -inducing proteins obtained from peritoneal exudates and/or spleens of mammals given cysteine protease.

Further, the present invention is cellular- and humoral-immunosuppression -inducing proteins against an antigen, which is obtained from spleens of mammals given cysteine protease and said antigen.

Further, the present invention is cellular- and humoral-immunosuppression -inducing proteins obtained by culturing macrophages contained in peritoneal exudates of mammals, in a medium containing cysteine protease.

Further, the present invention is a process for producing immunosuppression -inducing proteins, wherein peritoneal exudates and/or spleen cell suspensions from mammals given cysteine protease are purified using a column of hydrophilic vinyl polymer beads.

Further, the present invention is a process for producing immunosuppression -inducing proteins against an antigen, wherein spleen cell suspensions from mammals given cysteine protease and the antigen are purified using a column of hydrophilic vinyl polymer beads.

Further, the present invention is a process for producing immunosuppression -inducing proteins, wherein macrophages contained in peritoneal exudates of mammals are cultured in a medium containing cysteine protease and the culture is then purified using a column of concanavalin-A-immobilized agarose and a column of hydrophilic vinyl polymer beads.

Hereinafter, the present invention is described in detail.

The immunosuppressive agent of the present invention comprises immunosuppression-inducing proteins as an active ingredient. This immunosuppression-inducing proteins includes those derived from (1) peritoneal exudates from mammals given cysteine protease, (2) peritoneal exudate macrophages of mammals, having been cultured in a medium containing cysteine protease, (3) spleens of mammals given cysteine protease, and (4) spleens of mammals given cysteine protease and antigen.

Cysteine protease that can be used in the present invention includes the one extracted from cells and tissues of animals ranging from invertebrates such as parasites to vertebrates such as mammals or cultured cells thereof, as well as the one from a gene recombinant of cysteine protease derived therefrom by genetic engineering means (recombinant DNA techniques), e.g. the method described in Japanese Patent Application Laid-Open Publication No. 304,956/93.

Preferably, cysteine protease is one with a higher content of acidic amino acids than basic amino acids and is active at a neutral pH range. Such cysteine protease includes one with the amino acid sequence of SEQ ID NO:1 in the Sequence Listing or those amino acid sequences of SEQ ID NO:1 where Ala at the 15-position from the N-terminal is replaced by Pro, Ser at the 21-position by Glu, Arg at the 58-position by Met, Val at the 59-position by Ala, Gln at the 61-position by Glu, Ala at the 69-position by Ser, and/or Tyr at the 77-position by Asp, as well as one with cysteine protease activity having those amino acid sequence of SEQ ID NO:1 where one or more amino acids are added, deleted and/or replaced.

In the case of (1) above, 4 to 5 days before administration of cysteine protease to mammals, stimulators such as oyster glycogen, thioglycolate medium, sodium caseinate, etc., can be injected into their peritoneal cavities to yield more peritoneal exudate cells from which peritoneal exudate macrophages are recovered. For use, cysteine protease is dissolved preferably in physiological saline (referred to hereinafter as saline). Although the dosage of cysteine protease varies depending on the type of mammal to be used, it is preferably in the range of about 30 to about 100 µg/kg, and cysteine protease is administered preferably by injection.

About 0.25 to 0.5 day after administration of cysteine protease, the peritoneal exudate is recovered. The peritoneal exudate can be recovered using a medium such as Hanks' solution etc. The supernatant obtained by centrifuging the exudate can be used as cellular and humoral immunosuppressive inducers (The immunosuppressive inducers mean crude immunosuppression-inducing proteins and can include immunosuppression-inducing proteins). Macrophages are contained in the peritoneal exudate, and an extract from the macrophages can also be used as cellular and humoral immunosuppression-inducing proteins.

In the case of (2) above, 4 to 5 days before recovery of macrophages from peritoneal exudates of mammals, stimulators such as oyster glycogen, sodium caseinate, mineral oil, starch plus peptone, thioglycolate medium etc. can be administered to the peritoneal cavities of mammals to exude the peritoneal exudate cells. The peritoneal exudate cells can be recovered by injecting Hanks' solution etc. to the peritoneal cavities of mammals to wash their peritoneal cavities.

To obtain macrophages from the peritoneal exudate, the peritoneal exudate is centrifuged to recover cells. Then, the cells are cultured in a prescribed medium, and the nonadherent cells are removed, and the adherent cells are rinsed. These procedures are repeated 5 to 6 times.

Then, the macrophages thus obtained are cultured in the presence of cysteine protease. As the medium used, mention may be made of Cosmedium-001 medium containing penicillin (100 µg/ml) and streptomycin (100 µg/ml) in addition to cysteine protease (1 µg/ml). Its composition is shown in Table 1.

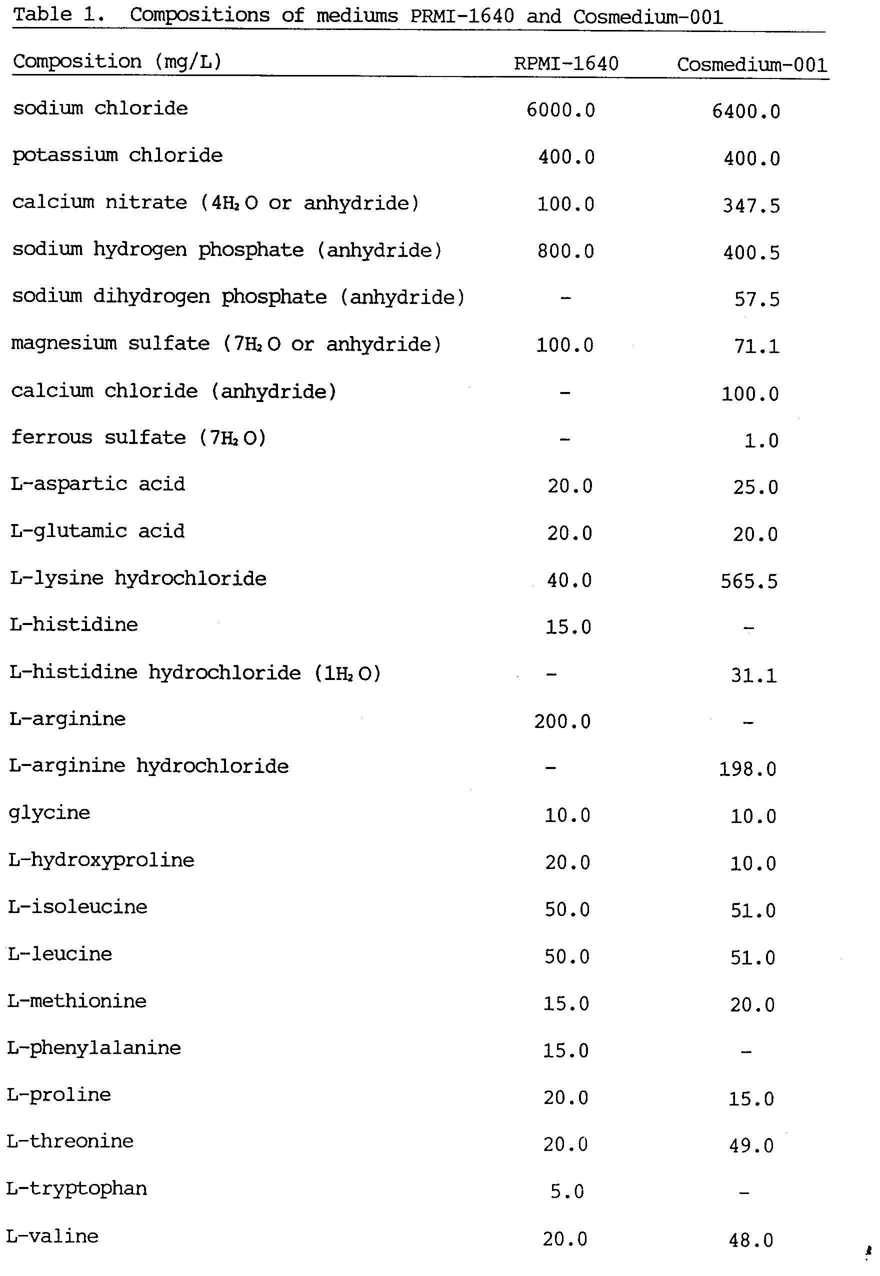

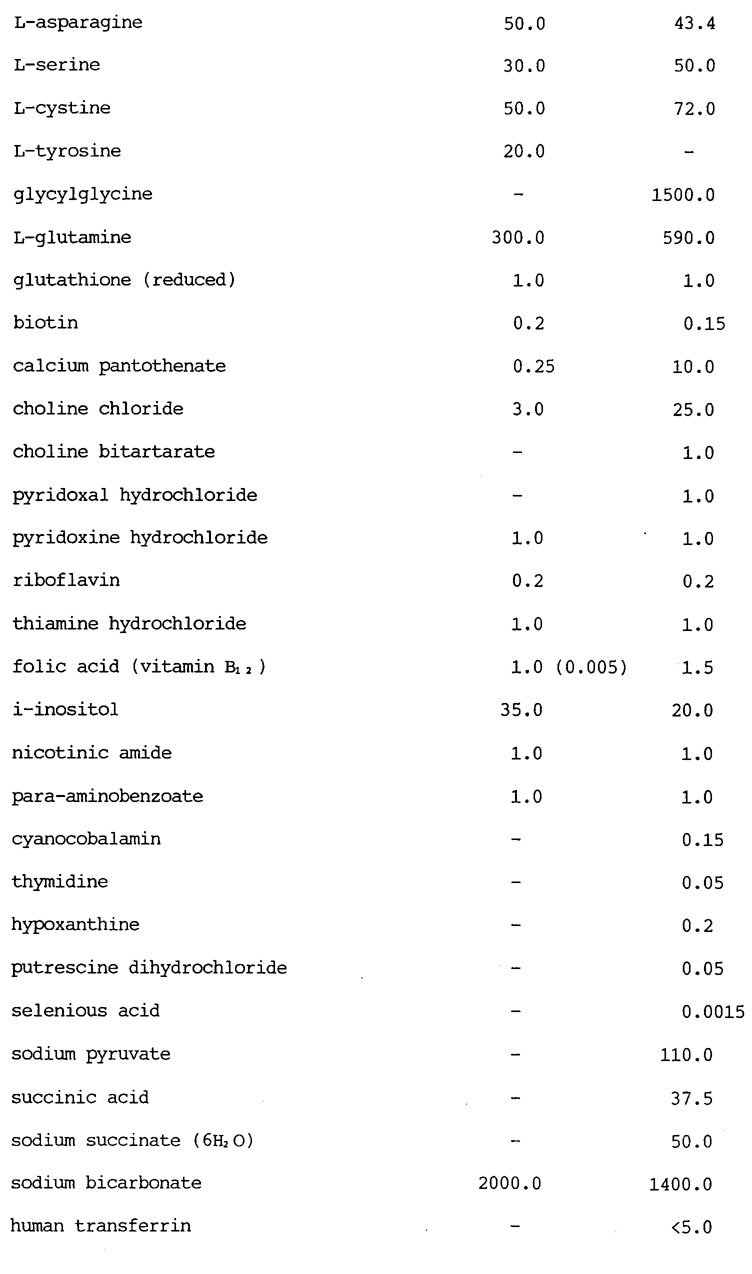

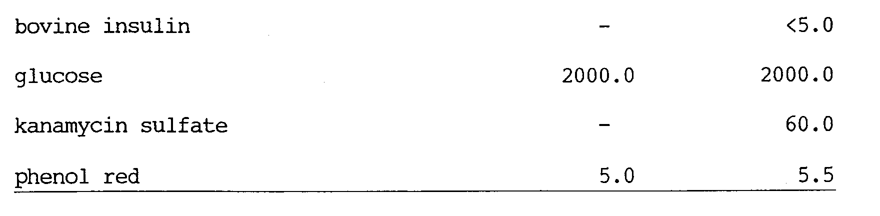

After 3 to 6 hours in culture, the culture is centrifuged. The supernatant thus obtained can be used for the fractionation of cellular and humoral immunosuppression-inducing proteins.

For purification, the supernatant is dialyzed at 3.5 to 4.5 °C for about 15 to 16 hours against TBS-7.2 (10 mM Tris-HCl buffer, pH 7.2, 0.15 M sodium chloride) containing e.g. 1 mM Ca²⁺ and 1 mM Mn²⁺, and the dialyzate is centrifuged at 3.5×10⁴ to 4.5×10⁴ x g at 3.5 to 4.5 °C for 20 to 30 minutes. The supernatant thus obtained is applied to a column of concanavalin A-agarose equilibrated with e.g. the above buffer. The non-adsorbed materials are washed out with the above buffer and the adsorbed materials are eluted with the buffer containing e.g. 0.1 M methylmannose.

Their fractions are preferably concentrated and dialyzed against a buffer such as TBS-7.2 for 4 to 6 hours. Then, the dialyzate is applied to a column equilibrated with PBS containing e.g. 0.01 % sodium azide, preferably a column packed with hydrophilic vinyl polymer beads, e.g. a column packed with Toyopearl HW-55s (Tosoh, Japan). A high molecular weight fraction of 190 kDa as an elution protein peak from the column is separated from a low molecular weight fraction of 19 kDa. The respective fractions are concentrated and dialyzed against a buffer e.g. PBS etc. The high molecular weight fraction thus obtained is a humoral immunosuppression-inducing protein and the low molecular weight fraction is a cellular immunosuppression-inducing protein.
(3) For recovery of spleens, the spleens are excised from mammals 0.5 to 5 days after injection of cysteine protease into the peritoneal cavities, and they are suspended in Hanks' solution to prepare a spleen cell suspension, followed by centrifugation. The supernatant thus obtained can be used to be fractionated into a cellular or humoral immunosuppressive protein. For purification, 0.5 day after injection of cysteine protease, the spleens are homogenized in phosphate buffered saline (PBS) (pH 7.2 to 7.4) containing 0.01 % sodium azide, and the homogenate is centrifuged at 3.5×10⁴ to 4.5×10⁴ x g at 3.5 to 4.5 °C for 20 to 30 minutes, and the resulting supernatant is applied to a column equilibrated with the above buffer, preferably a column packed with hydrophilic vinyl polymer beads, e.g. a column packed with Toyopearl HW-55s (Tosoh). An elution protein peak of 82 to 26 kDa is collected and concentrated by e.g. ultrafiltration. Preferably, the eluate is further applied to a similar column and an elution peak of 45 to 60 kDa is collected, concentrated and dialyzed against a buffer such as PBS etc. The fraction thus obtained is the cellular immunosuppression-inducing protein.

Then, the spleens after 5 days after injection of cysteine protease are treated and applied to the column in the same manner as above. The second peak of 110 to 350 kDa is collected, concentrated and applied to a column packed with Toyopearl HW-55s equilibrated with the same buffer, and a protein peak with a molecular weight of 150 kDa is collected, concentrated and dialyzed against PBS-7.2. The fraction thus obtained is the humoral immunosuppression-inducing protein.

In the case of (4) above where cysteine protease and antigen are administered to mammals, a spleen cell suspension is prepared in the same manner as in (3) above, and the supernatant obtained by centrifuging the suspension can be used as a cellular or humoral immunosuppressive substance for the antigen.

Because the active ingredient of the present invention obtained in this manner can inhibit the production of antibody against antigen or transplanted tissues and induce immune tolerance, it is possible to prepare an extremely useful immunosuppressive agent by incorporation of this active ingredient.

Although the above active ingredient itself can be administered as the immunosuppressive agent of the present invention, it may be mixed in a usual manner with suitable base materials for pharmaceutical manufacturing. For example, the immunosuppressive agent of the present invention may be in the form of injection, inhalation, ointment, lyophilized product, etc., and its dosage varies depending on the form of administration and the object of treatment.

For example, the agent as an injection can be administered by subcutaneous, muscular or intravenous injection or by mixed fluid supplementation. The dosage of the agent as an injection ranges preferably from about 0.05 to 1.0 mg/kg.

The immunosuppression-inducing proteins obtained from the peritoneal cavities (macrophages) or spleens of mammals is contained as the active ingredient in the immunosuppressive agent of the present invention. However, the active ingredient may further be those obtained from cultured cells or genetic recombinants derived therefrom by genetic recombination techniques (recombinant DNA techniques) as well as those obtained by chemical synthesis.

If a mammal is injected in the peritoneal cavity or caudal vein with the immunosuppressive agent of the present invention during a period of several hours before or several days after administration of antigen, the production of cellular and humoral antibody against the antigen is inhibited and the cellular and humoral immunity is inhibited for a long period of time. If the immunosuppressive agent of the present invention is previously injected, there is the effect of preventing the onset of autoimmune disease. Further, if the immunosuppressive agent of the present invention is injected and skin tissues are transplanted as xenografts, the tissues can take well for a long time and immune tolerance toward the transplanted tissues can be induced.

Although the detailed mechanism of this immunosuppressive action is not completely elucidated, it is believed that the immunosuppressive agent of the present invention, when administered to a living body, lowers the ability of immunocytes such as macrophages, lymphocytes etc., and inhibits the expression of IL-2 receptor and an adherent factor, to inhibit antibody production and induce immune tolerance in the living body.

### BRIEF DESCRIPTION OF DRAWINGS

FIG. 1 shows experimental schemes (A to D) for immunosuppression by the present immunosuppression-inducing proteins.

FIG. 2 shows experimental schemes (E to H) for immunosuppression by the present immunosuppression-inducing proteins.

FIG. 3 is a graph showing the percentage of survival with time in MRL-lpr/lpr mice treated with the present immunosuppressive inducer (ISI-4).

FIG. 4 is a graph showing the preventive effect of the present immunosuppressive inducer (ISI-4) against nephritis in MRL-lpr/lpr mice.

### BEST MODE FOR CARRYING OUT THE INVENTION

The present invention is specifically described by reference to the following examples which are not intended to limit the scope of the present invention.

### (Example 1) Extraction of immunosuppressive inducers

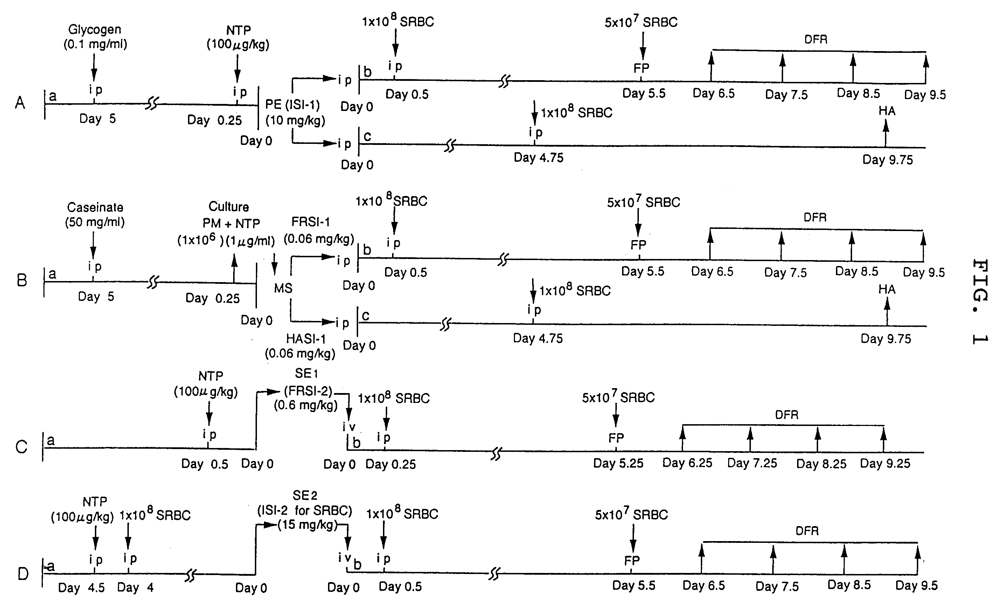

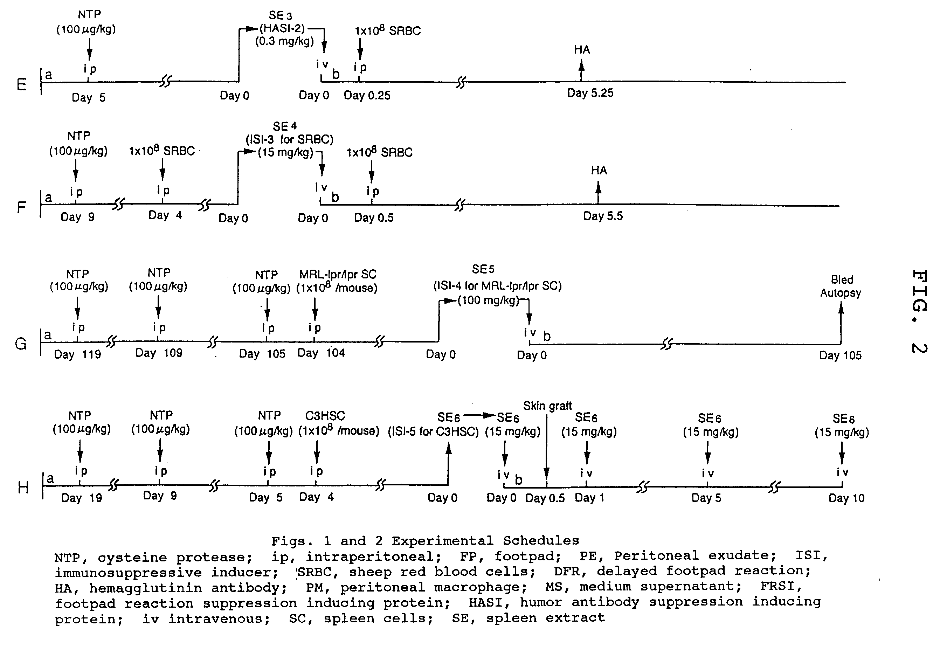

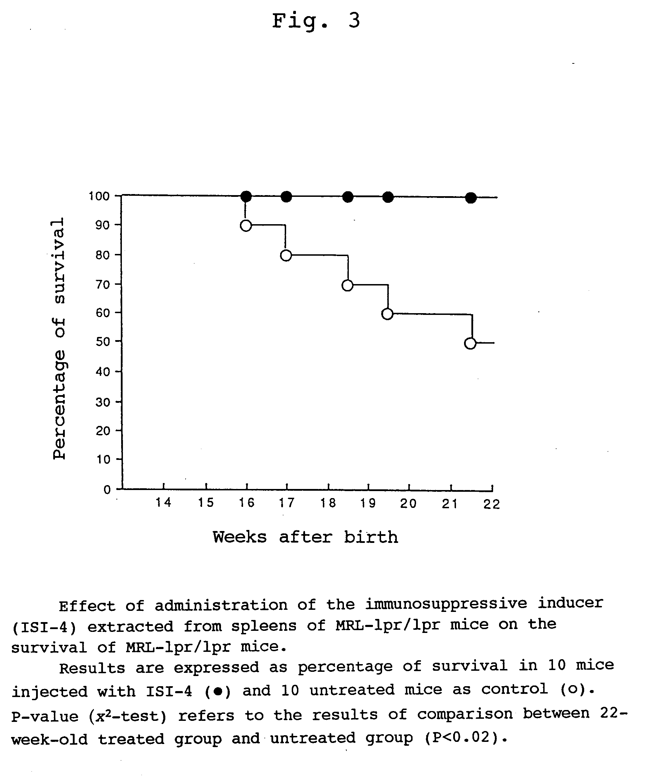

(A) Hartley strain female guinea pigs were injected intraperitoneally with 10 ml oyster glycogen (0.1 mg/ml) (Tokyo Kasei, Ltd.) in saline. 4.75 days after the injection, the animals were injected intraperitoneally with 3 ml cysteine protease (100 µl/kg) in saline which was extracted and purified according to the method described by Yamakami and Hamajima (Comp. Bioch. Phys. 87B, 643-648 (1987)). 6 hours after the injection, the peritoneal exudates were recovered using 10 ml Hanks' solution from the animals and centrifuged at 1000×g for 10 minutes, and the supernatant was used as cellular and humoral immunosuppressive inducer (ISI-1) (Fig. 1A, PE).
(B) Hartley strain female guinea pigs were injected intraperitoneally with 10 ml of 5 % sodium caseinate in saline. 4.5 days (4 to 5 days) after the injection, the peritoneal exudate cells were recovered from the animals by peritoneal lavage using 30 ml Hanks' solution containing heparin (10 units/ml). The cells were centrifuged and the precipitated cells were transferred at 1×10⁶ cells/ml to RPMI-1640 medium (for the composition, see Table 1). 5 ml of the cell suspension was put to a plastic vessel and incubated at 37 °C for 30 minutes in a 5 % CO₂ atmosphere. Then, the cells not adhered to the vessel were removed with pipetting, and the macrophages adhered to the vessel were rinsed with RPMI-1640 medium. The macrophages were purified by repeating these procedures 6 times, i.e. after 3 hours in culture.
   The macrophages were incubated at 1×10⁷ cells/ml for 6 hours in 10 ml Cosmedium-001 containing penicillin (100 µg/ml), streptomycin (100 µg/ml) and cysteine protease (1 µg/ml) (for the composition, see Table 1). Then, the cultured medium was centrifuged and the supernatant was used for the fractionation of cellular and humoral immunosuppression-inducing proteins (FRSI-1 and HASI-1) (Fig. 1B, MS).
(C) Female C3H/He mice were injected intraperitoneally with cysteine protease (100 µg/kg). The spleens were harvested from the animals 0.5 day after the injection and then minced gently in Hanks' solution, and the tissue fragments were squeezed with two glass slides to release cells into Hanks' solution, and also the suspensions were centrifuged. The supernatant was used for the fractionation of cellular immunosuppression-inducing protein (FRSI-2) (Fig. 1C, SE₁ ).
(D) Female C3H/He mice were injected intraperitoneally with cysteine protease (100 µg/kg) 4.5 days earlier and with 1×10⁸ sheep red blood cells (SRBC) 4 days earlier. The spleens were harvested from the mice and centrifuged. The supernatant was used as cellular immunosuppressive inducer (ISI-2) toward SRBC (Fig. 1D, SE₂ ).
(E) Female C3H/He mice were injected intraperitoneally with cysteine protease (100 µg/kg), and the spleens were harvested from the animals 5 days after the injection, then suspended and centrifuged. The supernatant was used for the fractionation of humoral immunosuppression-inducing protein (HASI-2) toward antibody production (Fig. 2E, SE₃ ).
(F) Female C3H/He mice were injected intraperitoneally with cysteine protease (100 µg/kg) 9 days earlier and with 1×10⁸ SRBC 4 days earlier. The spleens were harvested from the mice, suspended and centrifuged. The supernatant was used as humoral immunosuppressive inducer (ISI-3) toward SRBC (Fig. 2F, SE₄ ).
(G) Female MRL-lpr/lpr mice, 7 week-old, were injected intraperitoneally with cysteine protease (100 µg/kg) 119, 109, and 105 days earlier and with 1×10⁸ spleen cells from 22-week-old female MRL-lpr/lpr mice, 104 days earlier. The spleens of the mice were harvested, suspended and centrifuged. The supernatant was used as immunosuppressive inducer (ISI-4) toward the autoimmune disease of MRL-lpr/lpr mouse (Fig. 2G, SE₅ ).
(H) Female AKR mice were injected intraperitoneally with cysteine protease (100 µg/kg) 19, 9, and 5 days earlier and with 1×10⁸ spleen cells from female C3H/He mice, 4 days earlier. The spleens of the mice were harvested, suspended and centrifuged. The supernatant was used as immunosuppressive inducer (ISI-5) toward C3H/He mice (Fig. 2H, SE₆ ).

### (Example 2) Purification of immunosuppression-inducing proteins

(B') 20 ml culture supernatant obtained in Example 1(B) was dialyzed against TBS-7.2 (10 mM Tris-HCl buffer, pH 7.2, 0.15 M sodium chloride) containing 1 mM Ca²⁺ and 1 mM Mn²⁺ at 4 °C for 16 hours. The dialyzed culture was centrifuged at 40,000×g at 4°C for 10 minutes, and the resulting supernatant was applied to a concanavalin A-agarose column (1.2×4 cm) equilibrated with the same buffer. The non-adsorbed materials were washed out with the same buffer, and the adsorbed materials were eluted from the column with 0.1 M methylmannose in the buffer.
   The eluted fraction was concentrated to about 1 ml and dialyzed against TBS-7.2 for 6 hours. The concentrated protein was applied to a Toyopearl HW-55s (Tosoh) column (1.5×48 cm) equilibrated with PBS (phosphate buffered saline) containing 0.01 % sodium azide. As a result, it was separated into fractions ranging from a high molecular weight fraction of 190 kDa to a low molecular weight fraction of 19 kDa. Each fraction was concentrated to 0.5 ml and dialyzed against PBS. These purified high and low molecular weight immunosuppression-inducing proteins were designated HASI-1 and FRSI-1, respectively.
(C') The supernatant (spleen crude extract) obtained in Example 1(C) was homogenized in PBS-7.2 (phosphate buffered saline, pH 7.2) containing 0.01 % sodium azide and then centrifuged at 40,000×g at 4°C for 20 minutes. The resulting supernatant (10 ml) was applied to a column (2.5×40 cm) of Toyopearl HW-55s (Tosoh) equilibrated with the same buffer. The elution protein peak from the column, which has a molecular weight ranging from 82 kDa to 26 kDa, was collected and concentrated to 2.5 ml by ultrafiltration. The fraction thus obtained was further purified by gel filtration on a column (1.5×47 cm) of Toyopearl HW-55s equilibrated with the same buffer, and the elution peak from the column, which has a molecular weight ranging from 45 kDa to 60 kDa, was collected. This fraction was concentrated to 0.5 ml and dialyzed against PBS-7.2. This purified immunosuppression-inducing protein was designated FRSI-2.
(E') The supernatant (spleen crude extract) obtained in Example 1(E) was homogenized in PBS-7.2 containing 0.01 % sodium azide and then centrifuged at 40,000×g at 4°C for 20 minutes. The resulting supernatant (10 ml) was applied to a column (2.5×40 cm) of Toyopearl HW-55s (Tosoh) equilibrated with the same buffer. The elution protein peak from the column, which has a molecular weight ranging from 110 kDa to 350 kDa, was collected and concentrated to 2.5 ml by ultrafiltration. The fraction thus obtained was further purified by gel filtration on a column (1.5×48 cm) of Toyopearl HW-55s equilibrated with the same buffer, and the elution protein peak from the column, which has a molecular weight of around 150 kDa, was collected. This fraction was concentrated to 0.5 ml and dialyzed against PBS-7.2. This purified immunosuppression-inducing protein was designated HASI-2.

### (Experimental Example 1) Experiment of cellular-immunity inhibition

Female C3H/He mice were injected intraperitoneally with the immunosuppressive inducer ISI-1 (10 mg/kg) and 0.5 day later with 0.1 ml saline containing 1×10⁸ SRBC as antigen for immunization. 5 days after the immunization, the animals were injected in the footpad with 0.05 ml saline containing 5×10⁷ SRBC, to elicit the response after the initial immunization. The swelling (thickness) of the footpad was measured 24, 48, 72 and 96 hours later (Fig. 1A, b). The results are shown in Table 2.

In addition to this, female C3H/He mice were injected intraperitoneally with the immunosuppression-inducing protein FRSI-1 (0.06 mg/kg) and measured for the swelling of the footpad (Fig. 1B, b) in the same manner as above. The results are shown in Table 2.

Further, female C3H/He mice were injected in the caudal vein with the immunosuppression-inducing protein FRSI-2 (0.6 mg/kg) and 0.25 day later in the peritoneal cavity with 0.1 ml saline containing 1×10⁸ SRBC as antigen for immunization. 5 days after the immunization, the animals were injected in the footpad with 0.05 ml saline containing 5×10⁷ SRBC, to elicit the response after the initial immunization. The swelling (thickness) of the footpad was measured 24, 48,72 and 96 hours later (Fig. 1C, b). The results are shown in Table 2.

Further, female C3H/He mice were injected in the caudal vein with the immunosuppression-inducing protein ISI-2 (15 mg/kg) and 0.5 day later in the peritoneal cavity with 0.1 ml saline containing 1×10⁸ SRBC as antigen for immunization. Thereafter, the animals were injected with SRBC and examined for the swelling of the footpad (Fig. 1D, b) in the same manner as above. The results are shown in Table 2.

As the control group, normal female C3H/He mice not given the immunosuppressive inducer or protein were injected with SRBC and examined for the swelling of the footpad (untreated mice as control) in same manner as above. The results are shown in Table 2.

**Table 2**

| Delayed footpad reaction (DFR) against SRBC in C3H female mice after the treatment with some inducers from peritoneal exudate macrophages and spleen of animals treated with cysteine protease | | | | | |
|---|---|---|---|---|---|
| Treatment of inducer (dose) | DFR (units = 0.1 mm) after | | | | P-value (t-test) |
| | 24 hrs | 48 hrs | 72 hrs | 96 hrs | |
| None control | 6.5±0.2 | 3.7±0.2 | 2.0±0.2 | 1.0±0.1 | - |
| ISI-1 (10 mg/kg) | 2.0±0.2 | 1.1±0.2 | 0.5±0.2 | 0.2±0.1 | <0.001 |
| FRSI-1 (0.06 mg/kg) | 2.5±0.2 | 1.3±0.2 | 0.7±0.2 | 0.3±0.1 | <0.001 |
| FRSI-2 (0.6 mg/kg) | 2.2±0.3 | 1.3±0.1 | 0.6±0.1 | 0.3±0.1 | <0.001 |
| ISI-2 (15 mg/kg) | 2.2±0.2 | 1.4±0.2 | 0.7±0.2 | 0.3±0.1 | <0.001 |
| Results are expressed as the mean±SE of 6 animals. | | | | | |

As can be seen from Table 2, the DFR (swelling) in the group given the immunosuppressive inducers or proteins is inhibited significantly (P<0.001), indicating that the present immunosuppressive inducers and proteins inhibit cellular immunity (i.e. delayed type hypersensitivity reaction) against SRBC.

### (Experimental Example 2) Experiment of inhibition of humoral-antibody production

Female C3H/He mice were injected in the peritoneal cavity with the immunosuppressive inducer ISI-1 (10 mg/kg) and 4.75 days later with 0.1 ml saline containing 1×10⁸ SRBC. 5 days after the injection, the animals were injected in the footpad with 0.05 ml saline containing 5×10⁷ SRBC as a booster. Antibody titer (HA) in serum against SRBC was measured 5, 10 and 15 days after the first administration of the antigen (Fig. 1A, c). The results are shown in Table 3.

In addition to this, other mice were injected intraperitoneally with the immunosuppressive inducer HASI-1 (0.06 mg/kg) and SRBC and then examined for the antibody titer (HA) in serum against SRBC (Fig. 1B, c) in the same manner as above. The results are shown in Table 3.

Further, female C3H/He mice were injected in the caudal vein with the immunosuppressive inducer HASI-2 (0.3 mg/kg) and 0.25 day later in the peritoneal cavity with 0.1 ml saline containing 1×10⁸ SRBC. 5 days after the injection, the animals were injected in the footpad with 0.05 ml saline containing 5×10⁷ SRBC as a booster. Antibody titer (HA) in serum against SRBC was measured 5, 10 and 15 days after the first administration of the antigen (Fig. 2E, b). The results are shown in Table 3.

Further, female C3H/He mice were injected in the caudal vein with the immunosuppressive inducer ISI-3 (15 mg/kg) and 0.5 day later in the peritoneal cavity with 0.1 ml saline containing 1×10⁸ SRBC. 5 days after the injection, the animals were injected in the footpad with 0.05 ml saline containing 5×10⁷ SRBC as a booster. Antibody titer (HA) in serum against SRBC was measured 5, 10 and 15 days after the first administration of the antigen (Fig. 2F, b). The results are shown in Table 3.

As the control group, normal female C3H/He mice not given the immunosuppressive inducer or protein were injected with SRBC and examined for the antibody titer (HA) in serum (untreated mice as control) in same manner as above. The results are shown in Table 3.

**Table 3**

| Humoral-antibody production against SRBC in C3H female mice after treatment with some inducers from peritoneal exudate macrophages and spleen of animals treated with cysteine protease. | | | | |
|---|---|---|---|---|
| Treatment of inducer (Dose) | Log2 of HA titer after | | | P-value (t-test) |
| | 5 days | 10 days | 15 days | |
| None control | 6.0±0.2 | 6.8±0.2 | 7.1±0.2 | - |
| ISI-1 (10 mg/kg) | 4.1±0.2 | 4.8±0.3 | 5.2±0.2 | <0.01 |
| HRSI-1 (0.06 mg/kg) | 4.0±0.3 | 4.8±0.3 | 5.0±0.2 | <0.01 |
| HRSI-2 (0.3 mg/kg) | 4.1±0.2 | 4.5±0.2 | 4.8±0.2 | <0.01 |
| ISI-3 (15 mg/kg) | 4.2±0.2 | 4.7±0.2 | 4.9±0.2 | <0.01 |
| Results are expressed as the mean±SE of 6 animals. | | | | |

As can be seen from Table 3, the antibody production in the group given the immunosuppressive inducers or proteins is inhibited significantly (P<0.01), indicating that the present immunosuppressive inducers and proteins inhibit humoral-antibody production against SRBC.

### (Experimental Example 3) Experiment of induction of immune tolerance

Female C3H/He mice, 12 week-old, were injected in the peritoneal cavity with the immunosuppression-inducing protein FRSI-1 (0.06 mg/kg) and 0.5 day later with 0.1 ml saline containing 1×10⁸ SRBC. 5 days thereafter, antibody titer (HA) in serum was measured, and each mouse was injected in the footpad with 0.05 ml saline containing 5×10⁷ SRBC for immunization and examined for the swelling of the footpad 24 hours later (first immunization). The results are shown in Table 4.

In addition to this, female C3H/He mice, 12 week-old, were injected in the caudal vein with the immunosuppression-inducing protein FRSI-2 (0.6 mg/kg) and 0.25 day later in the peritoneal cavity with 0.1 ml saline containing 1×10⁸ cells SRBC. Then, antibody titer (HA) was measured in the same manner, and each mouse was injected in the footpad with SRBC and examined for the swelling of the footpad 24 hours later (first immunization). The results are shown in Table 4.

Further, female C3H/He mice, 12 week-old, were injected in the peritoneal cavity with the immunosuppression-inducing protein HASI-1 (0.06 mg/kg) and 4.75 days later in the peritoneal cavity with 0.1 ml saline containing 1×10⁸ SRBC. Then, antibody titer (HA) was measured in the same manner, and each mouse was injected in the footpad with 0.05 ml saline containing 5×10⁷ SRBC and examined for the swelling of the footpad 24 hours later (first immunization). The results are shown in Table 4.

Further, female C3H/He mice, 12 week-old, were injected in the caudal vein with the immunosuppression-inducing protein HASI-2 (0.3 mg/kg) and 0.25 day later in the peritoneal cavity with 0.1 ml saline containing 1×10⁸ SRBC. Then, antibody titer (HA) was measured in the same manner, and each mouse was injected in the footpad with SRBC and examined for the swelling of the footpad 24 hours later (first immunization). The results are shown in Table 4.

3 months after the first immunization, the mice were injected again in the peritoneal cavity with 0.1 ml saline containing 1×10⁸ SRBC for immunization (second immunization). Then, the antibody titer (HA) in serum 5 days after the second immunization with SRBC was measured. Further, the mice were injected in the footpad with 0.05 ml saline containing 5×10⁷ SRBC for immunization and then examined for the swelling of the footpad 24 later. The results are shown in Table 4.

As the control group, normal female C3H/He mice not given the immunosuppressive inducer or protein (untreated mice as control) were injected with SRBC and examined for immune tolerance induction in the same manner as above. The results are shown in Table 4.

**Table 4**

| Delayed footpad reaction and antibody production against SRBC in C3H mice after treatment with some inducers from peritoneal exudate macrophages and spleens with animals treated with cysteine protease | | | | | |
|---|---|---|---|---|---|
| Treatment of inducer (Dose) | Immunization | DFR (units=0.1mm) after 24 hrs | P-value (t-test) | Log2 of HA titer after 5 days | P-value (t-test) |
| None control | first time* | 6.5±0.2 | ― | 6.0±0.2 | ― |
| | second time** | 7.0±0.2 | ― | 7.0±0.2 | ― |
| FRSI-1 (0.06 mg/kg) | first time | 2.5±0.2 | <0.001 | 5.5±0.3 | NS |
| | second time | 3.5±0.2 | <0.001 | 7.3±0.3 | NS |
| FRSI-2 (0.6 mg/kg) | first time | 2.2±0.2 | <0.001 | 5.7±0.2 | NS |
| | second time | 3.0±0.2 | <0.001 | 6.5±0.2 | NS |
| HASI-1 (0.06 mg/kg) | first time | 6.0±0.2 | NS | 4.0±0.3 | <0.01 |
| | second time | 7.0±0.2 | NS | 5.0±0.3 | <0.01 |
| HASI-2 (0.3 mg/kg) | first time | 5.5±0.3 | NS | 4.1±0.2 | <0.01 |
| | second time | 6.5±0.2 | NS | 5.0±0.2 | <0.01 |
| Results are expressed as the mean±SE of 6 animals. P-value: significant difference between the group given an immunosuppressive protein and the untreated group. *The first immunization: SRBC was administered as an antigen after injection of an immunosuppressive protein. **The second immunization: SRBC only was administered as an antigen 3 months after the first immunization. NS: no significant difference. | | | | | |

As can be seen from Table 4, there is the effect of inhibiting the immune reaction against SRBC even 3 months later (P<0.001) as compared with the control group, indicating that the present immunosuppression-inducing proteins induce immune tolerance. (Experimental Example 4) Experiment of the inhibition of the occurrence of autoimmune disease

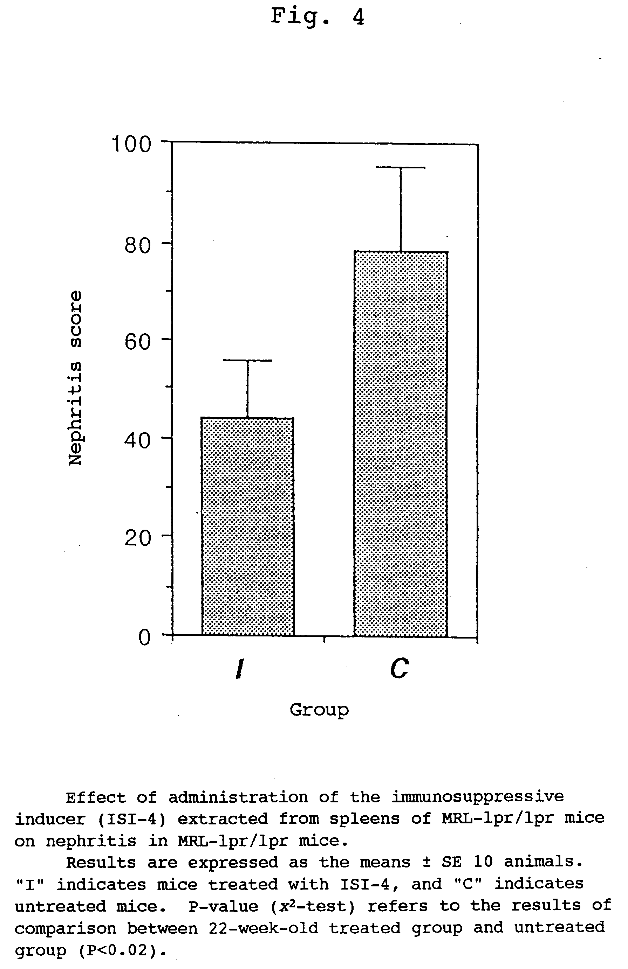

Female MRL-lpr/lpr mice, 7 week-old, were injected in the caudal vein with the immunosuppressive substance ISI-4 (100 mg/kg) and then examined for the percentage of survival (mortality) (Fig. 2G, b). The results are shown in Table 5 and Fig. 3. The MRL-lpr/lpr mice were further examined for their nephritis score. The results are shown in Fig. 4.

In addition to this, MRL-lpr/lpr mice not given the immunosuppressive inducer (untreated mice as control) were examined for the percentage of survival (mortality) in the same manner as above. The results are shown in Table 5 and Figs. 3 and 4.

**Table 5**

| Effect of spleen extract (ISI-4) from MRL-lpr/lpr mice on the mortality of MRL-lpr/lpr mice | | | |
|---|---|---|---|
| Treatment of inducer (Dose) | Mortality (22-week-old) | Percentage of diminution | P-value (χ ²-test) |
| None control | 5/10 | 50 | ― |
| ISI-4 (100mg/kg) | 0/10 | 0 | <0.02 |
| Results are expressed as percentage of 10 animals. | | | |

As is evident from Table 5 and Fig. 3, the percentage of survival of the group given the immunosuppressive inducer is higher (P<0.02) than that of the control group. Further, as is evident from Fig. 4, the nephritis score of the group given the immunosuppressive inducer is lower than that of the control group. Hence, it can be understood that the present immunosuppressive inducer can inhibit the occurrence of autoimmune disease.

### (Experimental Example 5) Experiment of immune inhibition of skin graft

Female AKR mice, 10 week-old, were injected in the caudal vein with the immunosuppressive inducer ISI-5 (15 mg/kg), and skin grafts from female C3H/He mice, 8 week-old, were transplanted to the animals 0.5 day after the injection. The animals were injected with the same immunosuppressive inducer Day 1, Day 5, and Day 10 after the injection of ISI-5, and the grafts were inspected for the degree of induration thereafter (Fig. 2H, b). The results are shown in Table 6.

In addition to this, skin grafts were transplanted in the same manner to female AKR mice not given the immunosuppressive inducer (untreated mice as control), and the grafts were inspected for the degree of induration. The results are shown in Table 6.

**Table 6**

| Effect of spleen extract (ISI-5) on the survival of C3H skin grafts in ARK mice | | | | | | | | |
|---|---|---|---|---|---|---|---|---|
| Recipient | Skin graft donors Day 0.5 | ISI-5 dose schedule (mg/kg) | | | | Skin graft survival time (days) individual mice | MST(day)±SE | P-value (t-tes) |
| | | Day 0 | Day 1 | Day 5 | Day 10 | | | |
| AKR(♀) | C3H(♀) | - | - | - | - | 14,14,16,18,18,20 | 16.7±0.9 | - |
| AKR(♀) | C3H(♀) | 15 | 15 | 15 | 15 | 21,23,27,30,35,43 | 29.8±3.0 | <0.05 |

As is evident from Table 6, the average survival time of the skin grafts (29.8±3.0 days) was longer than that of the control group (16.7±0.9 days), and the skin grafts could take well for a long period of time significantly (P<0.05).

The results of Experimental Examples 1 to 5 indicate that the immunosuppression-inducing proteins, when administered into a mouse via the peritoneal cavity or caudal vein before or after injecting antigen or transplanting tissues, can induce immunosuppression and immune tolerance against said antigen or tissues. Hence, the immunosuppressive agent containing the present immunosuppressive inducer as the active ingredient can be used to inhibit the production of antibody against antigen and transplanted tissues and induce immune tolerance in a living body.

The immunosuppression-inducing proteins used in Examples and Experimental Examples are those extracted from the peritoneal cavity (macrophage) or spleen. However, because the proteins cannot be obtained in a large amount from such materials and their isolation and purification require a long period and cost much, gene recombinants prepared from animal cultured cells by genetic engineering means (recombinant DNA techniques) are preferably used for large-scale production in order to practice the present invention in commercial scale.

### INDUSTRIAL APPLICABILITY

The immunosuppressive agent of the present invention can inhibit the antibody production against antigen or transplanted tissues, or induce immune tolerance, by administration to mammals. Hence, the immunosuppressive agent unlike conventional immunosuppressive agents, does not require administration for a long period of time to solve the problem side effects.

SEQUENCE LISTING

SEQUENCE ID NO: 1

(1) SEQUENCE LENGTH:  215

(2) SEQUENCE TYPE:  amino acid

(3) TOPOLOGY:  linear

(4) MOLECULAR TYPE:  peptide

(5) ORIGINAL SOURCE:

ORGANISM:  Paragonimus westermani metacecaria

(6) FEATURE:

(i)

LOCATION:  15

FEATURE KEY:  Xaa = Ala or Pro

(ii)

LOCATION:  21

FEATURE KEY:  Xaa = Ser or Glu

(iii)

LOCATION:  58

FEATURE KEY:  Xaa = Arg or Met

(iv)

LOCATION:  59

FEATURE KEY:  Xaa = Val or Ala

(v)

LOCATION:  61

FEATURE KEY:  Xaa = Gln or Glu

(vi)

LOCATION:  69

FEATURE KEY:  Xaa = Ala or Ser

(vii)

LOCATION: 77

FEATURE KEY: Xaa = Tyr or Asp

(7) SEQUENCE:

5 10

Ala Pro Glu Arg Ile Asp Trp Arg Ala Lys

15 20

Gly Ala Val Thr Xaa Val Glu Asn Gln Gly

25 30

Xaa Cys Gly Ser Cys Trp Ala Phe Ser Thr

35 40

Ala Gly Asn Val Glu Gly Gln Trp Phe Ile

45 50

Lys Thr Gly Gln Leu Val Ser Leu Ser Lys

55 60

Gln Gln Leu Val Asp Cys Asp Xaa Xaa Ala

65 70

Xaa Gly Cys Asn Gly Gly Trp Pro Xaa Ser

75 80

Ser Tyr Leu Glu Ile Met Xaa Met Gly Gly

85 90

Leu Glu Ser Glu Ser Asp Tyr Pro Tyr Val

95 100

Gly Val Glu Gln Thr Cys Ala Leu Asn Lys

105 110

Glu Lys Leu Val Ala Lys Ile Asp Asp Ser

115 120

Ile Val Leu Gly Pro Glu Glu Glu Asp His

125 130

Ala Ala Tyr Leu Ala Glu His Gly Pro Leu

135 140

Ser Thr Leu Leu Asn Ala Val Ala Leu Gln

145 150

Tyr Tyr Gln Ser Gly Val Leu Lys Pro Thr

155 160

Phe Glu Glu Cys Pro Asp Thr Glu Leu Asn

165 170

His Ala Val Leu Thr Val Gly Tyr Asp Lys

175 180

Glu Gly Asp Met Pro Tyr Trp Ile Ile Lys

185 190

Asn Ser Trp Gly Thr Asp Trp Gly Glu Lys

195 200

Gly Tyr Phe Arg Leu Phe Arg Gly Asp Cys

205 210

Thr Cys Gly Ile Asn Arg Met Ala Thr Ser

215

Ala Ile Ile Lys Lys

## Claims

1. An immunosuppressive agent comprising as an active ingredient an immunosuppression-inducing protein obtained from peritoneal exudates and/or spleens of mammals given cysteine protease.

2. An immunosuppressive agent comprising as an active ingredient an immunosuppression-inducing protein against an antigen, which is obtained from spleens of mammals given cysteine protease and said antigen.

3. The immunosuppressive agent according to claim 1, wherein the immunosuppression-inducing protein is obtained from macrophages contained in peritoneal exudates of mammals given cysteine protease.

4. An immunosuppressive agent comprising as an active ingredient an immunosuppression-inducing protein obtained by culturing macrophages contained in peritoneal exudates of mammals, in a medium containing cysteine protease.

5. A cellular-immunosuppression-inducing protein obtained from peritoneal exudates and/or spleens of mammals given cysteine protease.

6. A humoral-immunosuppression-inducing protein obtained from peritoneal exudates and/or spleens of mammals given cysteine protease.

7. A cellular-immunosuppression-inducing protein against an antigen, which is obtained from spleens of mammals given cysteine protease and said antigen.

8. A humoral-immunosuppression-inducing protein against an antigen, which is obtained from spleens of mammals given cysteine protease and said antigen.

9. A cellular-immunosuppression-inducing protein obtained by culturing macrophages contained in peritoneal exudates of mammals, in a medium containing cysteine protease.

10. A humoral-immunosuppression-inducing protein obtained by culturing macrophages contained in peritoneal exudates of mammals, in a medium containing cysteine protease.

11. A process for producing an immunosuppression-inducing protein, wherein peritoneal exudates and/or spleen cell suspensions from mammals given cysteine protease are purified using a column of hydrophilic vinyl polymer particles.

12. A process for producing an immunosuppression-inducing protein against an antigen, wherein spleen cell suspensions from mammals given cysteine protease and the antigen are purified using a column of hydrophilic vinyl polymer particles.

13. A process for producing an immunosuppression-inducing protein, wherein macrophages contained in peritoneal exudates of mammals are cultured in a medium containing cysteine protease and the culture is then purified using a column of concanavalin-A-immobilized agarose and a column of hydrophilic vinyl polymer particles.
